# EUROPEAN PATENT SPECIFICATION

(11) **EP 4 346 935 B1**
(45) Date of publication and mention of the grant of the patent: **22.04.2026**
(21) Application number: 22732064.5
(22) Date of filing: 30.05.2022
(51) Int. Cl.: A61L 33/00, A61L 33/06

(54) **COATING FOR MEDICAL DEVICES**
BESCHICHTUNG FÜR MEDIZINISCHE VORRICHTUNGEN
REVÊTEMENT POUR DISPOSITIFS MÉDICAUX

(30) Priority: 01.06.2021 GB 202107817
(43) Date of publication of application: 10.04.2024
(73) Proprietor: Smart Reactors Service Limited, Galway, H91 DCH9 (IE)
(72) Inventor: BRASSIL, Mark, Dublin Road, Galway, H91 DCH9 (IE)
(74) Representative: HGF
(86) International application number: PCT/EP2022/064659
(87) International publication number: WO 2022/253780

(56) References cited:
- US-A1- 2014 172 117
- US-A1- 2016 175 489
- BRYNDA E ET AL: "Albumin and heparin multilayer coatings for blood-contacting medical devices", JOURNAL OF BIOMEDICAL MATERIALS RESEARCH, WILEY, NEW YORK, NY, US, vol. 51, no. 2, 1 August 2000 (2000-08-01), pages 249 - 257, XP002370547, ISSN: 0021-9304, DOI: 10.1002/(SICI)1097-4636(200008)51:2<249::AID-JBM14>3.0.CO;2-X

## Description

### FIELD OF THE INVENTION

The invention is defined by the appended claims and relates to a coating for a medical device. The invention further relates to a medical device comprising the coating, and to uses and methods involving the coating or the medical device comprising the coating. The invention also relates to a method of manufacturing the coating and to a kit for coating a medical device.

### BACKGROUND

The formation of blood clots within an organ or a tissue can present a potentially life-threatening condition. When using a medical device, blood may come into contact with the foreign surfaces of the device, which may trigger the formation of a blood clot. Consequently, patients may be given an anticoagulant prior to the use of the medical device to suppress blood coagulation and the formation of blood clots. There are, however, considerable side effects associated with the administration of an anticoagulant that have to be taken into account. It may not be safe to administer such anticoagulants to some patients.

Some medical devices, such as stents, have been coated with heparin, which is an anticoagulant that prevents the formation of blood clots.

A coating comprising heparin and albumin is disclosed in Brynda et al., Journal of Biomedical Materials and Research (2000), vol 51, pages 249-257. Heparin is a glycosaminoglycan. It is can be difficult to coat onto a medical device and it is relatively expensive. The use of heparin is also associated with several side effects, which include bleeding, severe pain (e.g. at the injection site), nausea and unusual tiredness. There are also serious side effects associated with the use of heparin, such as heparin-induced thrombocytopenia. Heparin is contraindicated for several conditions, which include brain operation, operation on the spine, eye surgery, haemophilia, a deficiency of anti-clotting agents, significant uncontrolled high blood pressure, subacute infection of heart valve, a haemorrhage in the brain, bulge and tear of the aorta blood vessel wall, stomach or intestinal ulcer, ulcerative colitis, an inflammatory condition of the intestines, diverticulitis, severe liver disease, biliary and gallbladder problem, osteoporosis, and chronic kidney disease, which is stage 4 (severe) or stage 5 (failure).

Other types of coating have been applied to medical devices. These coatings typically comprise a multifunctional polymerizable compound. A problem with such coatings is that they can show poor coating performance and can degrade within a relatively short time. When the coatings lack mechanical robustness, the by-products of degradation can leach into the patient, which may be undesirable for certain types of coating.

### SUMMARY OF THE INVENTION

The references to the methods of treatment by therapy or surgery or in vivo diagnosis methods in this description are to be interpreted as references to coatings of the present invention for use in those methods.

The invention provides a coating for a medical device as defined in claims 1 to 9. The coating comprises a polymer layer. The polymer layer comprises a polymer having a plurality of hemocompatible groups. Each hemocompatible group is independently selected from a sulfonic acid group, a sulfonamide group, a sulfamic acid group, a hydrogen sulfate group and a conjugate base thereof.

The coating further comprises a base layer, and the polymer layer is disposed on the base layer. The base layer comprises a protein.

The coating of the invention is non-thrombogenic and is hemocompatible. The coating has a prophylactic effect in that it can prevent or inhibit the formation of blood clots, which, in turn, prevents or inhibits the onset, progression or recurrence of diseases and conditions associated with blood clots.

The inventors have discovered a polymeric material that has advantageous properties for use as a coating for a medical device, particularly when it includes hemocompatible groups. The hemocompatible groups may also be referred to herein as anti-thrombotic groups (i.e. the term "hemocompatible group" is synonymous with "anti-thrombotic group"). The hemocompatible groups contribute to the overall hemocompatibility of the coating.

The invention also provides a method of manufacturing a coating for a medical device as defined in claims 11 and 12. The method comprises (a) applying a polymer having a plurality of hemocompatible groups, as described herein, onto a base layer to form a polymer layer. Each hemocompatible group may be independently selected from a sulfonic acid group, a sulfonamide group, a sulfamic acid group, a hydrogen sulfate group and a conjugate base thereof. The base layer comprises a protein.

Also disclosed is a coating obtained or obtainable from the method of manufacturing a coating as described herein.

Also provided by the invention is a medical device having a surface coated with a coating as described herein.

A further aspect of the invention relates to a kit for coating a medical device as defined in claim 13. The kit comprises: (a) a polymer having a plurality of hemocompatible groups, as described herein; (b) a base coating solution; and (c) a coupling agent. Each hemocompatible group is independently selected from a sulfonic acid group, a sulfonamide group, a sulfamic acid group, a hydrogen sulfate group and a conjugate base thereof. The base coating solution comprises a protein.

The invention further relates to uses of the coating and the medical device for the uses as defined in the appended claims.

The coating of the invention or the medical device of the invention can be used in the treatment of the human or animal body by surgery or therapy and/or in a diagnostic method practised on the human or animal body. The diagnostic method practised on the human or animal body is typically an *in vivo* diagnostic method.

The coating of the invention or the medical device of the invention is for use in reducing or preventing the clotting of blood.

The disclosure also provides a method of reducing or preventing the clotting of blood. The method comprises contacting a medical device of the invention with blood.

An aspect of the method disclosed may be an *in vitro* method of reducing or preventing the clotting of blood, such as when processing the blood. The method may comprise contacting the medical device with blood, wherein the blood has been removed from a human or animal body. The blood may be contacted with the medical device to process the blood. The processed blood may not be returned to the human or animal body, preferably the human or animal body from which the blood was removed.

Also disclosed is a method of the invention is a method of reducing or preventing the clotting of blood in a diagnostic procedure. The method may comprise contacting the medical device with blood to obtain diagnostic information.

### DEFINITIONS

The term "biocompatible" as used herein, particularly in the context of the coating or its constituents, refers to the ability of a medical device or a material to perform with an appropriate host response in a specific application (e.g. as set out in ISO 10993-1 (2018)). The definition of "biocompatibility", its associated terms and tests for its evaluation in ISO 10993-1 (2018) are incorporated herein by reference. In general terms, biocompatibility refers to the ability of a medical device or a material to perform its desired function with respect to a medical therapy, without eliciting any undesirable local or systemic effects in the recipient or beneficiary of that therapy, but generating the most appropriate beneficial cellular or tissue response in that specific situation, and optimising the clinically relevant performance of that therapy.

The term "alkyl" as used herein refers to a straight or branched hydrocarbon chain radical consisting of carbon and hydrogen atoms, and containing no unsaturation. A "C₁₋₆ alkyl" group contains one to six carbon atoms. Unless stated otherwise specifically in the specification, an alkyl group is unsubstituted or may be substituted by one or more substituents selected from hydroxy, C₁₋₆ alkoxy and halo (e.g. fluoro, chloro, bromo or iodo), which is preferably fluoro. It is preferred that the alkyl group is unsubstituted.

The term "alkoxy" as used herein refers to a radical bonded through an oxygen atom of the formula -O-alkyl, where the alkyl group is defined above.

The term "aryl" as used herein refers to a radical derived from an aromatic monocyclic or polycyclic hydrocarbon ring system by removing a hydrogen atom from a ring carbon atom. The aromatic monocyclic or polycyclic hydrocarbon ring system contains only hydrogen atoms and carbon atoms, where at least one of the rings in the ring system is fully unsaturated (i.e. it contains a cyclic, delocalized [4n+2] π-electron system in accordance with the Hückel theory). The ring system from which aryl groups may be derived include, for example, benzene, indane, indene, tetralin and naphthalene.

The term "aryl-alkyl" as used herein refers to a radical bonded to an alkyl group, such as defined above, which alkyl group is further substituted by (or bonded to) an aryl group as defined above. Examples of aryl-alkyl groups include benzyl (PhCH₂-) and phenylethyl. A benzyl group is an example of a C₆ aryl-C₁ alkyl group.

The term "alkylene" as used herein refers to a straight or branched divalent hydrocarbon chain, which consists of carbon and hydrogen atoms, and contains no unsaturation. Examples of preferred alkylene groups include -CH₂-, -CH₂-CH₂-, -CH₂-CH₂-CH₂-, -CH(CH₃)-, and -C(CH₃)₂-CH₂-. A "C₁₋₁₀ alkylene" group contains one to ten carbon atoms.

The term "alkenylene" as used herein refers to a straight or branched divalent hydrocarbon chain, which consists of carbon and hydrogen atoms, and contains at least one double bond. The alkene group may have a *cis* or a *trans* arrangement. By way of example, alkenylene groups include -CH=CH-, -CH₂-CH=CH-, -C(CH₃)=CH-CH₂-, -CH₂-C(CH₃)=CH-CH₂-, and -CH₂-CH=C=CH-CH₂-. A "C₂₋₁₀ alkenylene" group contains two to ten carbon atoms. When the alkenylene contains more than one double bond, then the double bonds may be conjugated or non-conjugated. It is preferred that the alkenylene group does not comprise an allene group. More preferably, it is preferred that the alkenylene group comprises a single double bond.

The term "alkene" as used herein refers to a straight or branched hydrocarbon chain radical group consisting of carbon and hydrogen atoms, and containing at least one carbon-carbon double bond.

The term "alkynylene" as used herein refers to a straight or branched divalent hydrocarbon chain, which consists of carbon and hydrogen atoms, and contains at least triple double bond. Examples of alkylene groups include -C=C-, -CH₂-C≡C-, -CH(CH₃)-C≡C-CH₂-, and -CH₂- C=C-C=C C(CH₃)=CH-CH₂-. A "C₂₋₁₀ alkynylene" group contains two to ten carbon atoms. When the alkynylene contains more than one triple bond, then the triple bonds may be conjugated or non-conjugated. Preferably, an alkynylene group does not include a double bond (e.g. carbon-carbon double bond). More preferably, the alkynylene group comprises a single triple bond.

The term "phenylene" as used herein refers to a divalent radical derived from benzene (e.g. -C₆H₄-). The divalent radical (e.g. a di-substituted benzene ring) may have an *ortho* arrangement of substituents, a *meta* arrangement of substituents or a *para* arrangement of substituents.

Unless stated otherwise specifically in the specification, each group mentioned above (e.g. alkyl, alkene, alkoxy, aryl, aryl-alkyl, alkylene, alkenylene, alkynylene, phenylene) is unsubstituted.

It should be understood that unless expressly stated to the contrary, any reference to the formula of a repeating unit, a monomer or a polymer (e.g. the terms "repeating unit represented by formula (A-1)", "repeating unit represented by formula (B-1)", "monomer of formula (a-1)" or "monomer of formula (b-1)") includes any and all repeating units, monomers or polymers, respectively, described by and/or with reference to the formula. It should also be understood that these terms encompass all stereoisomers, especially *cis* and *trans* isomers, as well as optical isomers, i.e. *R and S* enantiomers, of such repeating units, monomers or polymers, such as in substantially pure form or as a mixture of stereoisomers and/or optical isomers.

As used in the present disclosure, the term "comprises" has an open meaning, which allows other, unspecified features to be present. This term embraces, but is not limited to, the semi-closed term "consisting essentially of" and the closed term "consisting of". Unless the context indicates otherwise, the term "comprises" may be replaced with either "consisting essentially of" or "consists of". The term "consisting essentially of" may also be replaced with "consists of".

### DETAILED DESCRIPTION

The invention is defined by the appended claims and provides a coating for a medical device. The coating reduces or prevents the clotting of blood. The coating is typically an antithrombotic coating, such as an anticoagulant coating and/or an antiplatelet coating, preferably an antiplatelet coating. The coating is typically a hemocompatible coating.

Any reference to "blood" as used herein generally refers to the blood of a human or a non-human animal, such as a mammalian animal. The invention can be used in veterinary applications. The term "blood" preferably refers to the blood of a human.

Typically, the coating is biocompatible, preferably biocompatible and/or hemocompatible.

Generally, the coating comprises a surface layer. The surface layer is the topmost layer of the coating. Thus, the surface layer may be an outer surface layer of the coating or a coated medical device. In use, the surface layer of the coating comes into contact with blood.

The surface layer or the coating (e.g. as a whole) may be in the form of a gel, preferably a hydrogel. The hydrogel may provide the medical device with a lubricious surface.

In general, the surface layer is biocompatible.

The surface layer comprises, or may consist essentially of, the polymer having a plurality of hemocompatible groups, as described herein. The polymer coating of the invention is defined by the appended claims. When the coating comprises a total of two layers, then the surface layer is the polymer layer.

Each hemocompatible group is selected from a sulfonic acid group, a sulfonamide group, a sulfamic acid group, a hydrogen sulfate group and a conjugate base thereof. The structure of each group is shown below, where the squiggly line indicates the point of attachment of the group to the polymer, either directly or indirectly.

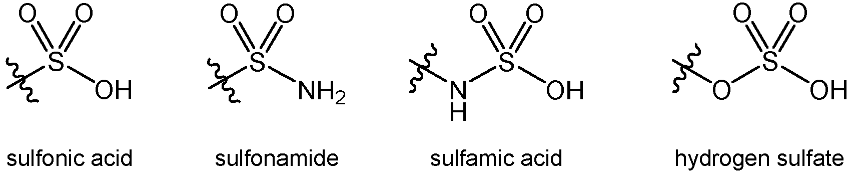

In general, it is preferred that each hemocompatible group is independently selected from a sulfonic acid group, a sulfamic acid group, a hydrogen sulfate group and a conjugate base thereof. More preferably, each hemocompatible group is independently selected from a sulfonic acid group or a sulfonate group (i.e. the conjugate base of a sulfonic acid group).

The conjugate base of each group is shown below. The conjugate base of each group refers to a sulfonate group, sulfonamide anion group, a sulfamate group or a sulfate group respectively.

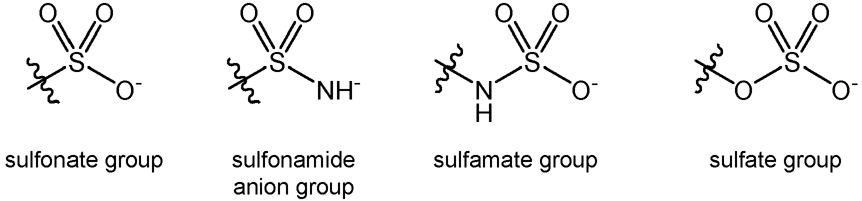

The plurality of hemocompatible groups is preferably the same hemocompatible group and/or a conjugate base thereof.

Typically, the plurality of hemocompatible groups is selected from a sulfonic acid group or a conjugate base thereof; a sulfonamide group or a conjugate base thereof; a sulfamic acid group or a conjugate base thereof; and a hydrogen sulfate group or a conjugate base thereof. More preferably, the plurality of hemocompatible groups is selected from a sulfonic acid group or a sulfonate group (i.e. the conjugate base of a sulfonic acid group).

Without wishing to be bound by theory, it is believed that when the hemocompatible groups described herein are ionised, then the negatively charged conjugate base (e.g. sulfonate group etc) repels the adhesion of platelets, thereby inhibiting or preventing the formation of a blood clot on a surface of the coating. The examples in International patent application no. PCT/EP2020/081383 demonstrate that the hemocompatible groups (referred to as anti-clotting groups in the International patent application) have activity in reducing or preventing blood clots. The polymer of the coating of the invention may provide a high density of hemocompatible groups because of the relatively small molecular size of the repeating unit.

The polymer comprising the plurality of hemocompatible groups has a polymer chain. The polymer chain is the backbone of the polymer. It is preferred that the polymer chain is biocompatible.

Generally, each hemocompatible group is attached or bonded to the polymer chain by a linker group. Thus, the polymer comprises a plurality of linker groups.

In principle, any linker group may be used to connect the hemocompatible group to the polymer chain. However, certain linker groups may provide, or contribute to, the advantageous properties of the coating.

It is believed that the linker group should be relatively short (e.g. 1 to 3 atoms in length) to ensure that the surface of the coating presents a high negative charge density to platelets. When longer linker groups are used, then it is possible for the surface of the coating to have a disordered arrangement of hemocompatible groups due to the conformational flexibility of the linker group. This can reduce the negative charge density provided by the surface of the coating.

In general, it is preferred that the linker group is biocompatible.

Each linker group covalently bonds a hemocompatible group to the polymer chain. Thus, the linker group may have a first end that is covalently bonded to the polymer chain and a second end that is covalently bonded to a hemocompatible group.

A single hemocompatible group may be attached or bonded by a single linker group to the polymer chain.

The polymer chain may be a linear polymer chain or a branched polymer chain. It is preferred that the polymer chain is a linear polymer chain.

The polymer or polymer chain typically has a number average (e.g. mean) molecular weight (Mn) of at least 300 g/mol, such as 300 to 50,000 g/mol, preferably 500 to 30,000 g/mol, more preferably 1,000 to 10,000 g/mol. The number average (e.g. mean) molecular weight (Mn) can be determined by light scattering.

The polymer having a plurality of hemocompatible groups comprises a repeating unit represented by formula (A 1): 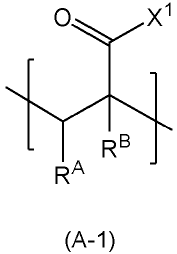 wherein:
R^{A} is selected from H and C₁₋₆ alkyl;
R^{B} is selected from H and C₁₋₆ alkyl;
X¹ is represented by formula (S-1):

   -Z¹-L¹-SO₂-Y¹ (S-1)
and wherein:
Z¹ is selected from O, NH and NR¹;
R¹ is selected from C₁₋₆ alkyl, C₆₋₁₀ aryl and C₆₋₁₀ aryl-C₁₋₆ alkyl;
Y' is selected from O⁻, OH, NH⁻ and NH₂;
L¹ is represented by formula (L-1):

   -P¹-Q¹-W¹- (L-1)
and wherein:
P¹ is selected from a single bond, C₁₋₁₀ alkylene, C₂₋₁₀ alkenylene, C₂₋₁₀ alkynylene and phenylene;
Q¹ is selected from a single bond, O, NH, NR² and phenylene;
W¹ is selected from a single bond, C₁₋₁₀ alkylene, C₂₋₁₀ alkenylene, C₂₋₁₀ alkynylene and phenylene; and
R² is selected from C₁₋₆ alkyl, C₆₋₁₀ aryl and C₆₋₁₀ aryl-C₁₋₆ alkyl.

The repeating unit represented by formula (A-1) may be a first repeating unit.

For convenience, the term "first repeating unit", particularly the reference to "first", is used as a label to distinguish this repeating unit from other types of repeating unit. The use of this term does not require the "first repeating unit" to be present when reference is made, for example, to a "second repeating unit" or to a "third repeating unit" etc. The same also applies to the terms "second repeating unit", "third repeating unit" etc.

The repeating unit represented by formula (A-1) above may, for example, be obtained or is obtainable from a monomer of formula (a-1) as described herein, such as 2-acrylamido-2-methyl-1-propane sulfonic acid (AMPS), 3-(acryloyloxy)propane-1-sulfonic acid or 3-(methacryloyloxy)-propane-1-sulfonic acid. These monomers are commercially available.

In formula (A-1), the moiety -L¹- represents the linker group.

In general, any layer, such as the polymer layer and/or the surface layer, that has a polymer comprising a repeating unit represented by formula (A-1) will typically comprise a polymer composition. The polymer composition has a distribution of molecules of the polymer, where each molecule of polymer includes a repeating unit (e.g. in varying numbers) as represented by formula (A-1) as described herein.

The repeating unit represented by formula (A-1) may be further represented by formula (A-2) below.

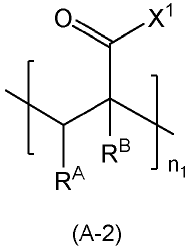

In formula (A-2), n₁ is an integer greater than 0, and each of R^{A}, R^{B} and X¹ are as defined herein.

In formula (A-1) or formula (A-2), it is preferred that R^{A} is selected from H and C₁₋₃ alkyl, preferably R^{A} is selected from H and methyl. Even more preferably, R^{A} is H.

In formula (A-1) or formula (A-2), it is preferred that R^{B} is selected from H and C₁₋₃ alkyl, preferably R^{B} is selected from H and methyl. Even more preferably, R^{B} is H.

In formula (A-1) or formula (A-2), when Z¹ is NR¹, then it is preferred that R¹ is C₁₋₆ alkyl, particularly methyl or ethyl.

Typically, Z¹, in formula (A-1) or formula (A-2), is preferably selected from O and NH. Z¹ may be O. More preferably, Z¹ is NH. When Z¹ is O or NH, then the linker group is attached to the polymer chain by an ester or amide group, which is typically a biocompatible group. Amide groups, in particular, are biocompatible because they are present in peptides.

P¹ is typically selected from a single bond, C₁₋₁₀ alkylene and phenylene; and W¹ may be selected from a single bond, C₁₋₁₀ alkylene and phenylene. More preferably, P¹ may be selected from a single bond and C₁₋₁₀ alkylene; and W¹ may be selected from a single bond and C₁₋₁₀ alkylene.

Alternatively, P¹ is selected from C₁₋₁₀ alkylene, C₂₋₁₀ alkenylene and C₂₋₁₀ alkynylene; Q¹ is selected from O, NH and NR² and W¹ is a single bond. More preferably Q¹ is selected from O and NH. In this arrangement, the hemocompatible group is a sulfamate group, a sulfate group or a conjugate base thereof.

Typically, for L¹ it is preferred that at least one of P', Q¹ and W¹ is not a single bond.

When Q¹ is O, it is generally preferred that P¹ is not a single bond. More preferably, when Q¹ is O, it is preferred that P¹ is C₁₋₁₀ alkylene, more preferably P¹ is C₂₋₄ alkylene.

When Q¹ is O and P¹ is not a single bond, it may be preferable that W¹ is not a single bond. More preferably, when Q¹ is O, it is preferred that P¹ is C₁₋₁₀ alkylene and W¹ is C₁₋₁₀ alkylene, more preferably P¹ is C₂₋₄ alkylene and W¹ is C₂₋₄ alkylene.

When Q¹ is phenylene, it is generally preferred that P¹ is a single bond or C₁₋₁₀ alkylene and W¹ is a single bond or C₁₋₁₀ alkylene. More preferably, when Q¹ is phenylene, it is preferred that P¹ is a single bond or C₁₋₃ alkylene and W¹ is a single bond or C₁₋₃ alkylene.

In general, it is preferred that P¹ is selected from C₁₋₁₀ alkylene, C₂₋₁₀ alkenylene and C₂₋₁₀ alkynylene; Q¹ is a single bond and W¹ is a single bond. More preferably, P¹ is C₁₋₁₀ alkylene, particularly C₂₋₆ alkylene, such as C₂₋₅ alkylene. Even more preferably P¹ is butylene, propylene or ethylene, preferably iso-butylene.

When P¹ is ethylene and both Q¹ and W¹ are single bonds, then the moiety represented by (S-1) can be formed from taurine.

The repeating unit represented by formula (A-1) or formula (A-2) may be anionic, such as when Y' is O⁻ or NH⁻. When the polymer chain is anionic, then Na⁺ or K⁺ may be present as a counter cation.

It is preferred that Y' is selected from O⁻, OH and NH₂. More preferably, Y' is selected from O⁻ and OH.

Typically, the integer represented by n₁ is at least 5, preferably at least 10. For example, n₁ may be from 5 to 500, such as from 10 to 300, more preferably from 15 to 100.

The polymer having a plurality of hemocompatible groups may comprise a repeating unit represented by formula (B-1): 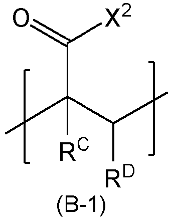 wherein:
R^{C} is selected from H and C₁₋₆ alkyl;
R^{D} is selected from H,C₁₋₆ alkyl and -C(O)X²;
each X² is independently selected from O⁻, OH, OR³, NH₂, NHR³ and N(R³)₂; and
each R³ is independently selected from optionally substituted C₁₋₆ alkyl, optionally substituted C₆₋₁₀ aryl and optionally substituted C₆₋₁₀ aryl-C₁₋₆ alkyl.

The expression "C(O)" represents a carbonyl group, i.e. C=O.

The repeating unit represented by formula (B-1) may be a second repeating unit.

The repeating unit represented by formula (B-1) above may, for example, be obtained or is obtainable from a monomer of formula (b-1) or a monomer of formula (b-2) as described herein, such as acrylic acid, methacrylic acid, an ester of acrylic acid, an ester of methacrylic acid, an amide of acrylic acid, an amide of methacrylic acid, maleic acid or an ester, amide or anhydride thereof, or maleimide. The ester of methacrylic acid may be 2-hydroxyethyl methacrylate (HEMA) or 2-hydroxypropyl methacrylate. These monomers are commercially available.

Generally, any layer, such as the polymer layer and/or the surface layer, that has a polymer comprising a repeating unit represented by formula (B-1) will typically comprise a polymer composition. The polymer composition has a distribution of molecules of the polymer, where each molecule of polymer includes a repeating unit (e.g. in varying numbers) as represented by formula (B-1) as described herein.

The repeating unit represented by formula (B-1) may be further represented by formula (B-2) below.

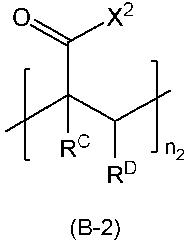

In formula (B-2), n₂ is an integer greater than 0, and each of R^{C}, R^{D} and X² are as defined herein.

In formula (B-1) or formula (B-2), it is preferred that R^{C} is selected from H and C₁₋₃ alkyl, preferably R^{C} is selected from H and methyl.

When R^{D} is selected from H and C₁₋₆ alkyl, then preferably R^{C} is methyl.

When R^{D} is -C(O)X², then preferably R^{C} is hydrogen.

In formula (B-1) or formula (B-2), it is preferred that R^{D} is selected from H, C₁₋₃ alkyl and -C(O)X²; preferably R^{D} is selected from H, methyl and -C(O)X². Even more preferably, R^{D} is hydrogen or -C(O)X². R^{D} may be hydrogen. R^{D} may be -C(O)X².

When R^{D} is -C(O)X², then the repeating unit represented by formula (B-1) or formula (B-2) contains two groups represented by X². Each X² may be the same or different. It is preferred that each X² is the same.

Generally, in formula (B-1) or formula (B-2), when X² is OR³, NHR³ or N(R³)₂, then each R³ is independently selected from optionally substituted C₁₋₆ alkyl, optionally substituted C₆₋₁₀ aryl and optionally substituted C₆₋₁₀ aryl-C₁₋₆ alkyl. Thus, each R³ may be independently selected from unsubstituted C₁₋₆ alkyl, substituted C₁₋₆ alkyl, unsubstituted C₆₋₁₀ aryl, substituted C₆₋₁₀ aryl, unsubstituted C₆₋₁₀ aryl-C₁₋₆ alkyl and substituted C₆₋₁₀ aryl-C₁₋₆ alkyl.

The C₁₋₆ alkyl, C₆₋₁₀ aryl and the C₆₋₁₀ aryl-C₁₋₆ alkyl may each independently be substituted with one or more substituents selected from hydroxy, C₁₋₆ alkoxy and halo.

It is preferred that each R³ is independently selected from hydroxy-substituted substituted C₂₋₆ alkyl, unsubstituted C₁₋₆ alkyl, optionally substituted C₆ aryl and optionally substituted C₆ aryl-C₁₋₆ alkyl. More preferably, R³ is independently selected from hydroxy-substituted substituted C₂₋₆ alkyl and unsubstituted C₁₋₆ alkyl, such as methyl or ethyl.

When X² is N(R³)₂, then each R³ may be the same or different. It is preferred that each R³ is the same.

The repeating unit represented by formula (B-1) or formula (B-2) may be anionic, such as when X² is O⁻. When the polymer chain is anionic, then Na⁺ or K⁺ may be present as a counter cation.

In formula (B-1) or formula (B-2), it is preferred that X² is selected from O⁻, OH, OR³, NH₂ and NHR³. More preferably, X² is selected from O⁻, OH, OR³ and NH₂. X² may selected from O⁻, OH and OR³. Alternatively, X² is selected from O⁻, OH and NH₂. Even more preferably, X² is selected from O⁻ and OH.

Typically, the integer represented by n₂ is at least 5, preferably at least 10. For example, n₂ may be from 5 to 500, such as from 10 to 300, more preferably from 15 to 100.

When the polymer having a plurality of hemocompatible groups comprises a repeating unit (e.g. a first repeating unit) represented by formula (A-1) and a repeating unit (e.g. a second repeating unit) represented by formula (B-1), then preferably (a) and/or (b) applies.
(a) R^{A} is different (e.g. is a different group) to R^{D}.
(b) R^{B} is different (e.g. is a different group) to R^{C}.

For example, when R^{A} is R^{A} is selected from H and C₁₋₆ alkyl, then R^{D} may be -C(O)X².

In general, it is preferred that when R^{B} is H, then R^{C} is C₁₋₆ alkyl, preferably C₁₋₃ alkyl, more preferably methyl.

Typically, the polymer having a plurality of hemocompatible groups is a copolymer.

The polymer having a plurality of hemocompatible groups comprises, or consists essentially of, (i) a first repeating unit represented by formula (A-1) and (ii) a second repeating unit represented by formula (B-1). Thus, the polymer is a copolymer comprising, or consisting essentially of, (i) a first repeating unit represented by formula (A-1) and (ii) a second repeating unit represented by formula (B-1).

When the polymer having a plurality of hemocompatible groups is a copolymer, then the copolymer may be an alternating copolymer, a random copolymer or a block copolymer. The copolymer may be an alternating copolymer. The copolymer may be a block copolymer. It is preferred that the copolymer is a random copolymer.

When the polymer having a plurality of hemocompatible groups is a random copolymer, then the random copolymer comprises, or consists essentially of, (i) a first repeating unit represented by formula (A-1) and (ii) a second repeating unit represented by formula (B-1). The repeating units are randomly distributed in the copolymer (e.g. within the polymer chain of each copolymer molecule).

When the polymer having a plurality of hemocompatible groups is an alternating random copolymer, then the alternating copolymer comprises, or consists essentially of, (i) a first repeating unit represented by formula (A-1) and (ii) a second repeating unit represented by formula (B-1). For example, the alternating copolymer may comprise, or consist essentially of, alternating first and second repeating units, preferably regularly alternating first and second repeating units (e.g. -(A-1)-(B-1)-(A-1)-(B-1)-...).

The alternating copolymer may comprise, or consist essentially of, a repeating unit represented by formula (1),

-[-(A-1)-(B-1)-]ₙ- (1)

wherein:
(A-1) is a repeating unit of formula (A-1) as described herein;
(B-1) is a repeating unit of formula (B-1) as described herein; and
n is an integer greater than 0.

When the polymer having a plurality of hemocompatible groups is a block copolymer, then the block copolymer comprises, or consists essentially of, (i) a first repeating unit represented by formula (A-1) and (ii) a second repeating unit represented by formula (B-1).

For example, the block copolymer may comprise, or consist essentially of, a repeating unit represented by formula (2),

-[(A-1)]n₁-[(B-1)]n₂- (2)

wherein:
(A-1) is a repeating unit of formula (A-1) as described herein;
(B-1) is a repeating unit of formula (B-1) as described herein;
n₁ is an integer greater than 0, such as described herein; and
n₂ is an integer greater than 0, such as described herein.

In an embodiment, the polymer having a plurality of hemocompatible groups does not comprise a repeating unit obtained or obtainable from a monomer of maleic acid or maleimide. In particular, the polymer having a plurality of hemocompatible groups does not comprise a repeating unit as defined in claim 4 and represented by formula (A-1) in the International publication of International patent application no. PCT/EP2020/081383.

Methods for preparing the polymer having a plurality of hemocompatible groups are known in the art.

The coating of the invention comprises a base layer. The base layer is for contact with, or disposal on, a surface of a medical device.

The base layer may be disposed on a surface of the medical device. The base layer is typically directly disposed on a surface of the medical device. Thus, the base layer is in direct contact with the surface.

It is generally preferred that the base layer adheres or bonds, preferably adheres, to a surface of the medical device.

Typically, the base layer is biocompatible.

The base layer is typically disposed under the polymer layer or a surface layer.

A polymer layer or the surface layer is, in general, directly disposed on the base layer. Thus, a polymer layer or the surface layer is typically coated onto the base layer.

In general, the surface layer is as defined in the appended claims.

When the coating comprises a total of two layers (e.g. only two layers), then the surface layer is the polymer layer. The polymer layer is disposed on the base layer.

The coating may comprise a plurality of layers disposed on the base layer. One of the plurality of layers is the surface layer. In addition to the surface layer, the plurality of layers may comprise one or more polymer layers, such as described herein, and/or one or more protein layers.

Generally, each protein layer comprises, or consists essentially of, a protein, such as a protein as described herein.

It is preferred that the plurality of layers is one or more pairs of layers, wherein each pair of layers consists essentially of a polymer layer and a protein layer. When there is one or more pairs of layers, then it is preferred that a polymer layer from a pair of layers is disposed on the base layer. The surface layer is preferably disposed on a protein layer from a pair of layers.

For example, the coating may comprise a base layer, a first polymer layer disposed on the base layer, a first protein layer disposed on the first polymer layer, a second polymer layer disposed on the first protein layer, a second protein layer disposed on the second polymer layer, and a surface layer disposed on the second protein layer. In this example, a first pair of layers is the first polymer layer and the first protein layer. A second pair of layers is the second polymer layer and the second protein layer.

In the present invention, the base layer comprises, or may consist essentially of, a protein, preferably a human protein.

The protein may be albumin. It is preferred that the albumin is a human albumin, preferably recombinant human albumin.

Albumin, particularly recombinant human albumin, can mimic the albumin found in the circulatory system of a human. It can adhere to a wide variety of surfaces, especially surfaces of medical devices, by means of a static attraction. It also provides a biopassive surface that can protect against platelet activation.

In the present invention, a polymer layer (or the surface layer when there is a total of two layers) is conjugated to the base layer.

When there is a plurality of layers, each polymer layer may be conjugated to an adjacent protein layer. The surface layer is conjugated to a protein layer.

When a polymer layer or the surface layer is conjugated to the base layer, then the polymer having a plurality of hemocompatible groups (e.g. from the polymer layer or the surface layer) is crosslinked to the protein (e.g. from the base layer).

Similarly, when a polymer layer is conjugated to an adjacent protein layer, then the polymer having a plurality of hemocompatible groups (e.g. from the polymer layer) is crosslinked to the protein (e.g. from the protein layer).

A repeating unit represented by formula (A-1) and/or a repeating unit represented by formula (B-1) may be coupled, preferably cross-linked, to the protein, such as to an amino acid of the protein. It is preferred that a repeating unit represented by formula (B-1) is coupled, preferably cross-linked, to the protein.

Conjugating the layers in this way, typically by forming cross-links between the polymer and the protein, is advantageous because the resulting coating is more robust. The coating can be used in acidic pH conditions without being degraded. The cross-links also improve adherence of the coating to a surface of the substrate, such as when alkaline conditions are used to apply the coating.

In general, the coating may further comprise an antimicrobial agent. The base layer and/or the surface layer may comprise the antimicrobial agent. The antimicrobial agent may, for example, be mixed with the components of the surface layer or the base layer.

The antimicrobial agent may comprise silver.

The coating of the invention is typically disposed on a surface of a medical device. The surface of the medical device may be an inner or an internal surface of the medical device (e.g. the inner surface inside a catheter) or an outer surface of the medical device. The coating may be disposed on a surface of the medical device that will come into with blood or a blood product containing platelets.

The coating may provide complete or partial coverage of the surface of the medical device.

In the coating of the invention, the base layer is disposed on a surface of the medical device. The base layer is typically directly disposed on a surface of the medical device. Thus, the base layer is in direct contact with the surface of the medical device.

The base layer may be directly applied to a surface of the medical device, particularly if it is able to adhere or bond to this surface.

The invention also provides a medical device. The medical device may be used where it comes into contact with blood or a blood product containing platelets.

The coating is disposed on a surface of the medical device. Thus, the medical device has a surface coated with the coating of the invention . For convenience, the surface coated with the coating of the invention is referred to as the "coated surface". The coated surface is for use in contact with blood or a blood product containing platelets, preferably blood.

Typically, the coating has a thickness of from 0.005 µm to 300 µm, preferably 0.05 µm to 200 µm (e.g. 0.1 µm to 200 µm), and more preferably 1 µm to 100 µm. Generally, the thickness of the coating is selected so that it does not significantly increase the profile of the medical device for use within a patient.

The medical device may be coated with a coating comprising, or consisting essentially of, the base layer and the surface layer. Alternatively, the medical device may be coated with the coating comprising a plurality of layers disposed on the base layer, as described herein. Thus, a surface of the medical device may be coated with several alternating layers of the protein layer and the polymer layer.

The medical device can be an implantable medical device or an extracorporeal medical device. The implantable medical device may be a permanently implantable medical device or a temporarily implantable medical device.

The medical device may be a transient blood contacting device, which may, for example, not be implantable or extracorporeal.

The medical device may be for *in vivo* use or for *in vitro* use. The medical device is preferably for *in vivo* use.

The medical device may have a component for *in vivo* use. It is preferred that at least a surface of the component is coated with the coating of the invention.

The coated surface of the medical device or the component of the medical device may be inserted into an area of a body, such as an afflicted area of the body. The area of the body may, for example, be an artery or a vein, such as a coronary artery or vein, a carotid artery or vein; a renal artery or vein; an iliac artery or vein; a femoral artery or vein; a popliteal artery or vein; a subclavian artery or vein; an intercranial artery or vein; the aorta; the vena cava; or a peripheral artery or vein.

Once in place, the coated surface prevents blood coagulation on and around the medical device or the component of the medical device, thereby inhibiting thrombosis, particularly sub-acute device thrombosis.

The medical device or component thereof may be a needle, catheter, a stent, a graft, a shunt, a dressing, a surgical staple, a guidewire, a cannula, a surgical instrument, an endoscope, an artificial organ or organoid (e.g. insulin secreting device), an implantable monitor or sensor, a defibrillator, a ventricular assist device, a pacemaker (e.g. cardiac pacemaker), an implantable pump (e.g. intra-aortic balloon pump or a ventricular assist pump), a cell reservoir (e.g. for stem cell placement), a prosthetic device (e.g. prosthetic heart valve), an orthopaedic device, an electrostimulation lead or lead tip, an implantable vascular access port, a blood storage bag, blood tubing, a breast implant, a pain management device, a prostate cancer treatment device, a dental implant, a focal epilepsy treatment device, a nerve regeneration conduit, a vena cava filter, a spinal repair device, a spinal cord stimulator, an internal hearing aid, a neuro aneurysm treatment device, a heart valve repair device, a intravitreal drug delivery device, a joint replacement, an ophthalmic implant, a blood oxygenator, a blood filter, a septal defect device, a haemodialysis unit, a hemoperfusion unit, a plasmapheresis unit or an anastomosis device.

Examples of catheters include a balloon or an inflation catheter, an injection catheter, a central venous catheter, an arterial catheter and an aspiration catheter. It may be preferable that the medical device is a central venous catheter or an aspiration catheter.

Examples of grafts include vascular grafts, stent grafts or bypass grafts. It may be preferable that the medical device is a bypass graft.

Examples of stents include a vascular stent, a urethra stent, a bile duct stent, a biliary stent, an oesophageal stent, and a tracheal or bronchial stent.

In general, it may be preferable that the medical device is selected from a central venous catheter, an aspiration catheter, a bypass graft, a perfusion line, a cardia bypass machine, and extracorporeal oxygenation and heat exchange circuitry.

The medical device or component thereof may comprise a metal, a polymeric material, glass or a ceramic. Thus, the coating of the invention may be coated onto a surface comprising a metal, a plastic or a ceramic.

The metal may, for example, be stainless steel, titanium, nickel, tantalum, cobalt, chromium, nickel, molybdenum, manganese, gold, platinum, iridium, silver, tungsten, a titanium alloy (e.g. nitinol), a nickel-chromium alloy (e.g. Inconel), a cobalt chromium alloy (e.g. elgiloy), a ferrous alloy, a palladium alloy, a rhenium alloy or a magnesium alloy.

The polymeric material may, for example, be cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, polyethylene or polytetrafluoroethylene.

The ceramic may, for example, include an oxide, a carbide, or a nitride of a transition metal, such as titanium oxides, hafnium oxides, iridium oxides, chromium oxides, aluminum oxides, and zirconium oxides.

The invention also provides a method of manufacturing the coating for a medical device.

An aspect of the invention is a method of coating a surface of medical device. This method is a method of manufacturing a coating for a medical device that is performed on a surface of the medical device.

Typically, the method comprises (a) applying a polymer having a plurality of hemocompatible groups as described herein onto a base layer (e.g. onto a base layer on a surface of a medical device). The polymer is applied onto the base layer to form a layer (e.g. a polymer layer or, when the coating comprises only two layers, then a surface layer).

The method of the invention is advantageous because the polymer having a plurality of hemocompatible groups is pre-formed before it is applied on to a base layer or a protein layer. This avoids the need to functionalise a precursor polymer with the hemocompatible groups *in situ,* such as when the precursor polymer has been applied onto a protein-containing layer. The formation of the polymer having a plurality of hemocompatible groups *in situ* when part of a layer restricts the chemical methodology that can be used because it may affect the protein-containing layer. This method permits a wider range of methodologies to be used to prepare the polymer.

The method of the invention allows a coating to be prepared having multiple layers, which can be built up layer-by-layer according to the required performance and cost. The layers can also be applied to a surface of the medical device without the need for solvents.

The method may comprise (a1) preparing a base layer as described herein on a surface of a medical device, then (a2) applying a polymer having a plurality of hemocompatible groups as described herein onto the base layer.

In step (a1) of the method, the base layer may be prepared by applying a base layer, such as by applying a base coating solution, to a surface of the medical device (e.g. to form a coated surface). After applying the base coating solution to the surface of the medical device, the coated surface is dried or is allowed to dry.

In general, the base coating solution comprises a protein as described herein. The base coating solution may be an aqueous solution, preferably a buffered aqueous solution, comprising the protein.

The base coating solution may have a pH of < 7, such as a pH from about 3.5 to about 6.5, preferably a pH of from about 4.0 to about 6.0.

The base coating solution may further comprise a stabiliser for the protein.

In principle, the base coating solution may be applied to a surface of the medical device using conventional techniques.

For example, the base coating solution can be applied to a surface of the medical device by, for example, dip-coating, spraying, washing, brushing, rollering, wiping, or spin coating. The base coating solution may be poured onto a surface of the medical device or the surface of the medical device may be soaked in or rinsed with the base coating solution. Alternatively, the base coating solution may be pumped through the medical device, such as to coat an inner surface of the medical device.

It is preferred that a surface of the medical device is soaked in, or rinsed with, the base coating solution.

The thickness of the base layer may be controlled by altering soaking time, flow rate etc and the number of coating steps. Each coating can be provided on a surface of the medical device as described herein in a series of applications. The number of applications may be selected to provide individual coated layers of suitable thickness, as well as a desired total number of multiple coatings, as desired.

The base layer is applied to a surface of the medical device before the surface layer.

Generally, step (a1) produces a base layer on a surface of the medical device.

Step (a1) may be repeated to produce the base layer. By repeating step (a1), the thickness of the base layer may be increased.

In step (a2), the polymer having a plurality of hemocompatible groups may be applied onto the base layer by applying a polymer coating dispersion onto the base layer (e.g. to form or provide a coated base layer). After applying the polymer coating dispersion onto the base layer, the coated base layer is dried or is allowed to dry.

In general, the polymer coating dispersion comprises the polymer having a plurality of hemocompatible groups and a liquid vehicle.

The polymer coating dispersion may be a solution or a suspension. Thus, the polymer may be dissolved in the liquid vehicle (e.g. when the polymer coating dispersion is a solution) or suspended in the liquid vehicle (e.g. when the polymer coating dispersion is a suspension).

The liquid vehicle may be water or ethanol. It is preferred that the liquid vehicle is water, preferably deionised water.

The polymer coating dispersion is preferably buffered.

The polymer coating dispersion may have a pH of < 7, such as a pH from about 3.5 to about 6.5, preferably a pH of from about 4.0 to about 6.0.

The polymer coating dispersion may be applied onto to the base layer using conventional techniques. For example, the polymer coating dispersion can be applied to a base layer (e.g. on a surface of the medical device) by, for example, dip-coating, spraying, washing, brushing, rollering, wiping, or spin coating. The polymer coating dispersion may be poured onto the base layer or the base layer on the surface of the medical device may be soaked in or rinsed with the polymer coating dispersion. Alternatively, the polymer coating dispersion may be pumped through the medical device, such as to coat the base layer on an inner surface of the medical device.

It is preferred that a surface of the medical device is soaked in, or rinsed with, the base coating solution.

The thickness of the polymer layer may be controlled by altering soaking time, flow rate etc and the number of coating steps.

It may be preferred that the base layer (e.g. on a surface of the medical device) is soaked in or rinsed with the polymer coating dispersion.

Generally, step (a2) produces a polymer layer on a protein layer, such as the base layer. When the coating comprises only two layers, then the polymer layer is the surface layer.

Step (a2) may be repeated to produce the polymer layer or the surface layer. By repeating step (a2), the thickness of the polymer layer or the surface layer may be increased.

When the coating comprises only two layers, then there is a single protein layer, which is the base layer, and a single polymer layer, which is the surface layer.

The coating may comprise a plurality of protein layers (e.g. including the base layer) and/or a plurality of polymer layers (e.g. including the surface layer).

After steps (a1) and (a2), the method may further comprise (a3) applying a protein layer as described herein to a polymer layer, then (a4) applying a polymer having a plurality of hemocompatible groups as described herein to the protein layer.

In step (a3) of the method, the protein layer may be prepared by applying the base coating solution as described herein to a polymer layer (e.g. to form a coated polymer layer surface). After applying the base coating solution to the polymer layer, the coated polymer layer surface is dried or is allowed to dry.

Step (a3) may be performed in the same way as step (a1). Thus, the base coating solution may be applied in the same way to the polymer layer as the base coating solution is applied to the surface of the medical device.

Generally, step (a3) produces a protein layer on the polymer layer.

Step (a3) may be repeated to produce the protein layer (e.g. before performing step (a4)). By repeating step (a3), the thickness of the protein layer may be increased.

In step (a4), the polymer having a plurality of hemocompatible groups may be applied onto the protein layer by applying a polymer coating dispersion as described herein onto the protein layer (e.g. to form or provide a coated protein layer). After applying the polymer coating dispersion onto the protein layer, the coated protein layer is dried or is allowed to dry.

Step (a4) may be performed in the same way as step (a2). Thus, the polymer coating dispersion may be applied in the same way to the protein layer as the polymer coating dispersion is applied to the base layer.

Generally, step (a4) produces a polymer layer on the protein layer.

Step (a4) may be repeated to produce the polymer layer. By repeating step (a4), the thickness of the polymer layer may be increased.

Both steps (a3) and (a4) may be performed once or repeated. It may be preferable to perform both steps (a3) and (a4) one, two or three times.

By performing steps (a3) and (a4), a coating having a plurality of layers may be manufactured, where the plurality of layers is one or more pairs of layers, wherein each pair of layers consists essentially of a polymer layer and a protein layer.

When step (a4) is performed for the final time, then the resulting polymer layer is the surface layer.

In general, the method of the invention comprises (b) coupling the polymer having a plurality of hemocompatible groups to the protein using a coupling agent. The coupling the polymer to the protein is preferably cross-linking the polymer to the protein.

The polymer may be cross-linked to the protein by forming amide bonds between the polymer and the protein. For example, the carboxylic acid, ester or amide in the repeating unit of formula (B-1) may form an amide bond with amino acids in the protein.

Typically, the coupling agent is a carbodiimide coupling agent. Carbodiimide coupling agents are known in the art.

The carbodiimide coupling agent may be selected from *N,N'-*dicyclohexylcarbodiimide (DCC), *N,N'*-diisopropylcarbodiimide (DIC) and 1-ethyl-3-(3-dimethylaminopropyl)carbodiimide (EDC). It is preferred that the carbodiimide coupling agent is EDC.

In step (b) of the method, the coupling the polymer having a plurality of hemocompatible groups to the protein using a coupling agent may be by applying a coupling agent solution to a coating comprising a protein layer as described herein and a polymer layer as described herein. The protein layer comprises the protein and the polymer layer comprises the polymer having a plurality of hemocompatible groups.

The coupling agent may be applied to the surface layer of the coating.

The polymer layer is the surface layer.

The protein layer is at least the base layer.

In general, the coupling agent solution comprises a coupling agent as described herein, such as a carbodiimide coupling agent.

The coupling agent solution may be an aqueous solution, preferably a buffered aqueous solution.

In principle, the coupling agent solution may be applied to the coating using conventional techniques.

The coupling agent solution may be applied onto to the base layer using conventional techniques. For example, the coupling agent solution can be applied to a polymer layer (e.g. on a surface of the medical device), such as the surface layer, by dip-coating, spraying, washing, brushing, rollering, wiping, or spin coating. The coupling agent solution may be poured onto the polymer layer (e.g. the surface layer) on the surface of the medical device may be soaked in or rinsed with the coupling agent solution. Alternatively, the coupling agent solution may be pumped through the medical device, such as to coat the polymer layer (e.g. the surface layer) on an inner surface of the medical device.

It is preferred that the coating is soaked in, or rinsed with, the coupling agent solution.

Generally, step (b) is performed to produce a crosslinked coating on a surface of the medical device. Before applying the coupling agent, the coating on the surface of the medical device is typically non-crosslinking (i.e. a polymer layer, such as the surface layer, is not conjugated to a protein layer, such as the base layer).

Step (b) may include repeatedly applying a coupling agent solution to the coating comprising the protein layer and the polymer layer. This may be to ensure sufficient cross-linking within the coating.

In the method of the invention, step (b) may be performed directly after step (a), such as directly after steps (a1) and (a2). Additionally or alternatively, step (b) may be performed after steps (a3) and (a4).

Step (b) may be performed after each repetition of steps (a3) and (a4). Alternatively, step (b) may be performed after all repetitions of steps (a3) and (a4) have been performed.

Typically, step (b) is the final step (e.g. the final step involving applying a solution) of the method.

Any remaining coupling agent solution can be removed, such as by rinsing with water.

In general, it is preferred that step (b) is only performed after the surface layer has been applied (e.g. the surface layer of a non-crosslinked coating). Thus, a single coupling step is performed once the layers have been applied to a surface of the medical device.

Another general feature of the method disclosed is that (a) may include preparing a polymer having a plurality of hemocompatible groups.

The polymer having a plurality of hemocompatible groups may be prepared by (i) polymerising a monomer of formula (a-1), 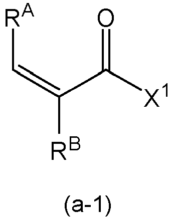 wherein R^{A}, R^{B} and X¹ are as described herein in relation to the repeating unit of formula (A-1).

The monomer of formula (a-1) may preferably be 2-acrylamido-2-methyl-1-propane sulfonic acid (AMPS), 3-(acryloyloxy)propane-1-sulfonic acid or 3-(methacryloyloxy)-propane-1-sulfonic acid. It is preferred that the monomer of formula (a-1) is 2-acrylamido-2-methyl-1-propane sulfonic acid (AMPS).

It is preferred that the polymer having a plurality of hemocompatible groups is prepared by (i) copolymerising a monomer of formula (a-1) as described herein with a monomer of formula (b-1) or (b-2), 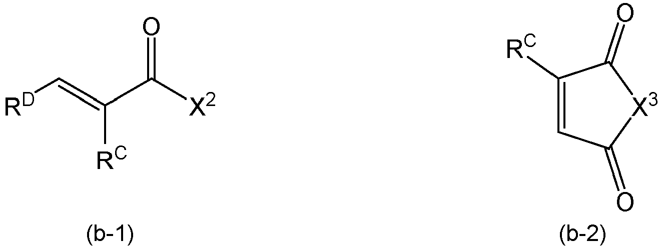 wherein R^{C}, R^{D} and X² are as described herein in relation to the repeating unit of formula (B-1); and X³ is selected from O, NH and NR³, wherein is R³ as defined herein. It is preferred that X³ is selected from O and NH, more preferably X³ is O.

The monomer of formula (b-1) may preferably be acrylic acid, methacrylic acid, an ester of acrylic acid, an ester of methacrylic acid, an amide of acrylic acid, an amide of methacrylic acid, maleic acid or an ester or an amide thereof. The ester of methacrylic acid may be 2-hydroxyethyl methacrylate (HEMA) or 2-hydroxypropyl methacrylate. The monomer of formula (b-2) may be maleic anhydride or maleimide.

It is preferred that the polymer having a plurality of hemocompatible groups is prepared by (i) copolymerising a monomer of formula (a-1) as described herein with a monomer of formula (b-1).

Typically, the copolymerisation is a free radical copolymerisation.

The copolymerisation, particularly the free radical copolymerisation, may be performed using methods known in the art.

In (i), the copolymerising a monomer of formula (a-1) with a monomer of formula (b-1) or formula (b-2) uses a copolymerisation catalyst.

When the copolymerisation is a free radical copolymerisation, then the copolymerisation catalyst may be a free radical initiator.

The free radical initiator may be any free radical initiator suitable for copolymerisation of the monomers. It is preferred that the free radical initiator is non-toxic to humans, preferably biocompatible.

The free radical initiator may, for example, be a thermal polymerisation initiator, a photopolymerisation initiator, a photocationic initiator or a photoanionic initiator.

The thermal polymerisation initiator may be an organic peroxide, an azo compound or a persulfate.

When the thermal polymerisation initiator is an organic peroxide, then the organic peroxide may be selected from *tert*-butylhydroperoxide (TBHP), cumene hydroperoxide, di-*tert*-butylhydroperoxide, dicumyl peroxide and benzoyl peroxide (BPO).

When the thermal polymerisation initiator is an azo compound, then the azo compound may be selected from azoisobutylnitrile (AIBN) and 2,2'-azobis[2-(2-imidazolin-2-yl)propane] dihydrochloride. It is preferred that the thermal polymerisation initiator is azoisobutylnitrile (AIBN).

When the thermal polymerisation initiator is a persulfate, then the persulfate may be selected from ammonium persulfate (also known as ammonium peroxydisulfate), sodium persulfate and potassium persulfate. It is preferred that the persulfate is ammonium persulfate.

Examples of the photopolymerisation initiator include (±)-camphorquinone, acetophenone, 4'-hydroxyacetophenone, 3'-hydroxyacetophenone, benzophenone, 3-methylbenzophenone, 2-methylbenzophenone, 3,4-dimethylbenzophenone, 3-hydroxybenzophenone, 4-hydroxyphenone, 4,4'-dihydroxybenzophenone, 4-benzoylbenzoic acid, 2-benzoylbenzoic acid, methyl 2-benzoylbenzoate, 4,4'-carbonyldiphthalic anhydride, 4-(dimethylamino)benzophenone, 4,4'-bis(dimethylamino)benzophenone, 4,4'-bis(diethylamino)benzophenone, 4,4'-dichlorobenzophenone, 4-phenylbenzophenone, 1,4-dibenzoylbenzene, 4-(p-tolylthio)benzophenone, dibenzosuberenone, benzil, p-anisil, methyl benzoylformate, 9,10-phenanthrenequinone, 2-benzoyl-2-propanol, 2-hydroxy-4'-(2-hydroxyethoxy)-2-methylpropiophenone, 1-benzoylcyclohexanol, benzoin, anisoin, benzoin methyl ether, benzoin ethyl ether, benzoin isopropyl ether, benzoin isobutyl ether, 2,2-diethoxyacetophenone, benzil dimethylketal, 2-methyl-4'-(methylthio)-2-morpholinopropiophenone, 2-benzyl-2-(dimethylamino)-4'-morpholiniobutyrophenone, 2-isonitrosopropiophenone, 9,10-phenantrenequinone, 2-ethylanthraquinone, sodium antraquinone-2-sulfonate monohydrate, 2-chlorothioxanthone, 1-chloro-4-propoxythioxanthone, 2-isopropylthioxanthone, 2,4-diethylthioxanthen-9-one and 2,7-dimethoxythioxanthone.

Examples of the photocationic initiator include diphenyliodonium trifluoromethanesulfonate, diphenyliodonium hexafluoroarsenate, diphenyliodonium hexafluorophosphate, triphenylsulfonium bromide, triphenylsulfonium tetrafluoroborate, triphenylsulfonium nonaflate, tri-*p*-tolylsulfonium triflate, tri-*p*-tolylsulfonium hexafluorophosphate, 4-nitrobenzenediazonium tetrafluoroborate, 2-(4-methoxyphenyl)-4,6-bis(trichloromethyl)-1,3,5-triazine, 2-(1,3-benzodioxol-5-yl)-4,6-bis(trichloromethyl)-1,3,5-triazine, 2-(4-methoxystyryl)-4,6-bis(trichloromethyl)-1,3,5-triazine, 2-(3,4-dimethoxystyryl)-4,6-bis(trichloromethyl)-1,3,5-triazine and 2-[2-(furan-2-yl)vinyl]-4,6-bis(trichloromethyl)-1,3,5-triazine.

Examples of the photoanionic initiator include acetophenone O-benzoyloxime, 2-nitrobenzyl cyclohexylcarbamate, 1,2-bis(4-methoxyphenyl)-2-oxoethyl cyclohexylcarbamate, 1-(2-formylbenzoyl)piperidine and nifedipine.

In general, it is preferred that the free radical initiator is a thermal polymerisation initiator, such as an azo compound or a persulfate.

The invention also provides a kit for coating a medical device. The kit comprises: (a) a polymer having a plurality of hemocompatible groups, as described herein; (b) a base coating solution, as described herein; and (c) a coupling agent.

The kit preferably comprises (a) a polymer coating dispersion, as described herein, which comprises the polymer having a plurality of hemocompatible groups.

Additionally or alternatively, the kit preferably comprises (c) a coupling agent solution as described herein, which comprises the coupling agent.

In general, the kit may comprise (d) instructions for preparing a coating on a surface of a medical device.

Also provided by the invention are the coating and the medical device for use as defined by the appended claims.

The coating or a medical device comprising the coating can be used in the treatment of the human or animal body by surgery or therapy and/or in a diagnostic method practised on the human or animal body.

The diagnostic method practised on the human or animal body is typically an *in vivo* diagnostic method.

The diagnostic method or the treatment by surgery or therapy typically involves the coating, or the medical device comprising the coating, coming into contact with blood.

The coating or a medical device comprising the coating is for use in reducing or preventing the clotting of blood, preferably reducing or preventing the clotting of blood on the coated surface of the medical device.

Also disclosed is a method of reducing or preventing the clotting of blood. The method comprises contacting a medical device of the invention with blood. The medical device is used in its normal manner, except that the presence of coating inhibits or prevents the formation of blood clots, particularly on the coated surface of the medical device.

One aspect of the method is an *in vitro* method. For example, the method may be an *in vitro* method of reducing or preventing the clotting of blood when processing the blood. The blood may be processed to produce processed blood or a blood product, such as a blood product for storage.

The method may comprise contacting the medical device with blood, wherein the blood has been removed from a human or animal body. The blood may be contacted with the medical device to process the blood.

The method may not include a step of administering the processed blood or the blood product to the human or animal body. The blood product or processed blood may not be returned to the human or animal body, preferably the human or animal body from which the blood was removed.

A further aspect of the method is a diagnostic method, particularly an *in vitro* and/or ex *vivo* diagnostic method.

The method may relate to a method of reducing or preventing the clotting of blood in a diagnostic procedure. The method may comprise contacting the medical device with blood to obtain diagnostic information. The medical device may be contacted with pre-obtained or pre-delivered blood.

The diagnostic procedure is not carried out on the human or animal body or the diagnostic procedure is carried out on a non-living (e.g. dead) human or animal body. Thus, the diagnostic method may not include a step of removing blood from a human or animal body, particularly a living human or animal body.

### Examples

The invention will now be illustrated by the following non-limiting examples.

### Example 1

### Preparation of AMPS-MAA copolymer

A round bottom flask equipped with a magnetic stirrer bar was charged with 2-acrylamido-2-methylpropane sulfonic acid (AMPS), deionised (DI) water, methacrylic acid (MAA) and azoisobutylnitrile (AIBN). The amount of each ingredient that was used is shown in Table 1 below.

The solution was stirred vigorously to dissolve the AMPS. A condenser (with bubbler) was fitted and the mixture sparged with nitrogen for 30 minutes. The nitrogen sparge was converted to a nitrogen sweep and the mixture heated to 70 °C for 24 hours. As the polymerisation proceeded the mixture took on a slightly hazy appearance. The mixture was cooled and the slightly hazy, moderately viscous solution was discharged.

**Table 1**

| **Batch No.** | **AMPS (g) [mmol]** | **MAA (g) [mmol]** | **AIBN (g) [mmol]** | **DI H₂O (9)** | **% Polymer** |
|---|---|---|---|---|---|
| 1 | 8.84 | 3.67 | 0.164 | 112.5 g | 10 % |
| | [43] | [43] | [1] | | |
| 2 | 10.62 | 1.89 | 0.164 | 112.5 g | 10 % |
| | [51] | [22] | [1] | | |
| 3 | 21.19 | 8.69 | 0.328 | 120.0 g | 20 % |
| | [103] | [103] | [2] | | |
| 4 | 25.49 | 4.51 | 0.328 | 120.0 g | 20 % |
| | [123] | [52] | [2] | | |

### Analytical data

The characterising data for each batch is shown in Table 2 below. In the NMR data, the intensity of the peaks is shown in bold.

**Table 2**

| **Batch No.** | **¹H NMR (298 K, D₂O) δ/ ppm** | **AMPS: MAA ratio** | **IR (Water Blank) cm-1** |
|---|---|---|---|
| 1 | 0.96 (br, s) CH₃, **6,** 1.41 (br, s) CH₃, **3,** 2.00 (br m) CH₂ **1, 2, 6,** 3.24 (br, m) CH₂ **4** | 0.97:1.00 | 1709 (Carboxylic C=O), 1647 (Amide C=O), 1451 (Amide C-N) |
| 2 | 0.94 (br, s) CH₃, **6,** 1.41 (br, s) CH₃, **3,** 2.01 (br m) CH₂ 1, **2, 6,** 3.24 (br, m) CH₂ **4** | 2.31:1.00 | 1712 (Carboxylic C=O), 1633 (Amide C=O), 1451 (Amide C-N) |
| 3 | 0.97 (br, s) CH₃, **6,** 1.43 (br, s) CH₃, **3,** 1.99 (br m) CH₂ **1, 2, 6,** 3.30 (br, m) CH₂ **4** | 1.13:1.00 | 1709 (Carboxylic C=O), 1637 (Amide C=O), 1451 (Amide C-N) |
| 4 | 0.97 (br, s) CH₃, **6,** 1.42 (br, s) CH₃, **3,** 2.01 (br m) CH₂ **1, 2, 6,** 3.29 (br, m) CH₂ **4** | 2.36:1.00 | 1718 (Carboxylic C=O), 1636 (Amide C=O), 1454 (Amide C-N) |

### Recombinant human albumin coating

A solution of recombinant human albumin (Albumedix^{™}) was prepared in a distilled water in a DI water buffer at a pH of 4.0 to 4.5 (via HCl & NaOH titration) to provide a concentration of the albumin in solution of approximately 0.05 g/2000 mL.

Samples of PVC tubing were washed with, or suspended in, the albumin solution at room temperature for 20 minutes to produce samples coated with human albumin.

All of the albumin coated samples were subsequently washed with, or soaked in, DI water for 10 minutes and then dried.

### Coating with AMPS-MAA copolymer

A solution of the AMPS-MAA copolymer from Batch No. 4 above was prepared using a DI water buffer at a pH of 4.0 to 4.5 (via HCl & NaOH titration) at a concentration of 0.2 mL per 1000 mL.

The samples coated with human albumin were washed with, or soaked in, the solution of the AMPS-MAA copolymer for 20 minutes.

After applying the AMPS-MAA copolymer coating, the coated samples were subsequently washed with, or soaked in, DI water for 10 minutes and then dried.

### Crosslinking the coatings

A solution of 1-ethyl-3-(3-dimethylaminopropyl)-carbodiimide (EDC) was prepared in a DI water buffer at a pH of 5.0 to 5.5 (via HCl & NaOH titration) to provide a concentration of 75 mg of EDC per 1000 mL.

The coated samples were washed for 20 minutes in the EDC solution. After washing in the EDC solution, the samples were subsequently washed with DI water for 30 minutes and then dried.

### Coated samples

Two sets of samples were prepared using the albumin and copolymer coating processes described above. A first set of samples had a single coating of human albumin and the AMPS-MAA copolymer, which are prepared as described above.

A second set of samples were prepared having a triple coating of each of the albumin and copolymer layers, where the layers were applied in an alternating arrangement. For the second set of samples, the process above of applying the albumin coating and then the copolymer coating was followed, and repeated for a total of three times.

Both the first set and the second set of samples were treated with toluidine blue. Toluidine blue has a high affinity for acidic groups, such as any sulfonic acid groups present in the coating. After the treatment, the coated samples showed a pink colour, which confirmed the presence of the sulfonic acid groups.

## Claims

1. A coating for a medical device comprising:
a base layer comprising a protein; and
a polymer layer disposed on the base layer;
wherein:
the polymer layer is conjugated to the base layer;
the polymer layer comprises a polymer having a plurality of hemocompatible groups, and the polymer having a plurality of hemocompatible groups is crosslinked to the protein;
each hemocompatible group is independently selected from a sulfonic acid group, a sulfonamide group, a sulfamic acid group, a hydrogen sulfate group and a conjugate base thereof, and wherein the polymer having a plurality of hemocompatible groups comprises a repeating unit represented by formula (A-1): 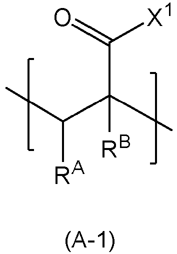
wherein:
R^{A} is selected from H and C₁₋₆ alkyl;
R^{B} is selected from H and C₁₋₆ alkyl;
X¹ is represented by formula (S-1):
-Z¹-L¹-SO₂-Y¹ (S-1)
wherein:
Z¹ is selected from O, NH and NR¹;
R¹ is selected from C₁₋₆ alkyl, C₆₋₁₀ aryl and C₆₋₁₀ aryl-C₁₋₆ alkyl;
Y' is selected from O⁻, OH, NH⁻ and NH₂;
L¹ is represented by formula (L-1):
-P¹-Q¹-W¹- (L-1)
wherein:
P¹ is selected from a single bond, C₁₋₁₀ alkylene, C₂₋₁₀ alkenylene, C₂₋₁₀ alkynylene and phenylene;
Q¹ is selected from a single bond, O, NH, NR² and phenylene; and
W¹ is selected from a single bond, C₁₋₁₀ alkylene, C₂₋₁₀ alkenylene, C₂₋₁₀ alkynylene and phenylene; and
R² is selected from C₁₋₆ alkyl, C₆₋₁₀ aryl and C₆₋₁₀ aryl-C₁₋₆ alkyl.

2. The coating according to claim 1, wherein the polymer having a plurality of hemocompatible groups is crosslinked to the protein by amide bonds formed between the polymer and the protein.

3. The coating according to claim 1 or claim 2, wherein the protein is albumin, preferably a human albumin.

4. The coating according to any one of the preceding claims, wherein each hemocompatible group is selected from a sulfonic acid group or a sulfonate group.

5. The coating according to any one of claims 1 to 4, wherein:
(a) R^{A} is selected from H and methyl, preferably R^{A} is hydrogen; and/or
(b) R^{B} is selected from H and methyl, preferably R^{B} is hydrogen.

6. The coating according to any one of claims 1 to 5, wherein:
(a) Z¹ is selected from O and NH, preferably Z¹ is NH; and/or
(b) Y' is selected from O⁻ and OH.

7. The coating according to any one of claims 1 to 6, wherein:
(a) P¹ is selected from C₁₋₁₀ alkylene, C₂₋₁₀ alkenylene and C₂₋₁₀ alkynylene; Q¹ is a single bond and W¹ is a single bond, preferably P¹ is C₁₋₁₀ alkylene, optionally C₂₋₆ alkylene, or more preferably P¹ is iso-butylene; or
(b) P¹ is selected from C₁₋₁₀ alkylene, C₂₋₁₀ alkenylene and C₂₋₁₀ alkynylene; Q¹ is selected from O, NH and NR² and W¹ is a single bond, preferably Q¹ is selected from O and NH.

8. The coating according to any one of the preceding claims, wherein the polymer having a plurality of hemocompatible groups comprises a repeating unit represented by formula (B-1): 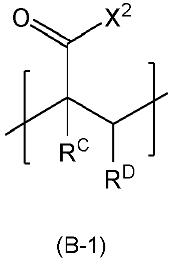 wherein:
R^{C} is selected from H and C₁₋₆ alkyl;
R^{D} is selected from H, C₁₋₆ alkyl and -C(O)X²;
each X² is independently selected from O⁻, OH, OR³, NH₂, NHR³ and N(R³)₂; and
each R³ is independently selected from optionally substituted C₁₋₆ alkyl, optionally substituted C₆₋₁₀ aryl and optionally substituted C₆₋₁₀ aryl-C₁₋₆ alkyl.

9. The coating according to any one of the preceding claims, wherein the polymer having a plurality of hemocompatible groups is a copolymer.

10. A medical device having a surface coated with a coating according to any one of claims 1 to 9.

11. A method of manufacturing a coating for a medical device as defined in any one of claims 1 to 9, the method comprising:
(a) applying a polymer having a plurality of hemocompatible groups onto a base layer to form a polymer layer,
wherein:
each hemocompatible group is independently selected from a sulfonic acid group, a sulfonamide group, a sulfamic acid group, a hydrogen sulfate group and a conjugate base thereof;
the base layer comprises a protein; and
the polymer having a plurality of hemocompatible groups comprises a repeating unit represented by formula (A-1): 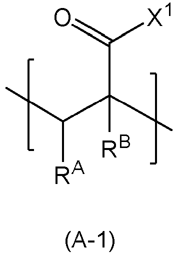
wherein:
R^{A} is selected from H and C₁₋₆ alkyl;
R^{B} is selected from H and C₁₋₆ alkyl;
X¹ is represented by formula (S-1):
-Z¹-L¹-SO₂-Y¹ (S-1)
wherein:
Z¹ is selected from O, NH and NR¹;
R¹ is selected from C₁₋₆ alkyl, C₆₋₁₀ aryl and C₆₋₁₀ aryl-C₁₋₆ alkyl;
Y' is selected from O⁻, OH, NH⁻ and NH₂;
L¹ is represented by formula (L-1):
-P¹-Q¹-W¹- (L-1)
wherein:
P¹ is selected from a single bond, C₁₋₁₀ alkylene, C₂₋₁₀ alkenylene, C₂₋₁₀ alkynylene and phenylene;
Q¹ is selected from a single bond, O, NH, NR² and phenylene; and
W¹ is selected from a single bond, C₁₋₁₀ alkylene, C₂₋₁₀ alkenylene, C₂₋₁₀ alkynylene and phenylene; and
R² is selected from C₁₋₆ alkyl, C₆₋₁₀ aryl and C₆₋₁₀ aryl-C₁₋₆ alkyl;
(b) coupling the polymer having a plurality of hemocompatible groups to the protein using a coupling agent.

12. The method of claim 11, wherein the coupling agent is a carbodiimide cross-coupling agent.

13. A kit for coating a medical device with a coating as defined in any one of claims 1 to 9, the kit comprising:
(a) a polymer having a plurality of hemocompatible groups, wherein each hemocompatible group is independently selected from a sulfonic acid group, a sulfonamide group, a sulfamic acid group, a hydrogen sulfate group and a conjugate base thereof,
(b) a base coating solution comprising a protein; and
(c) a coupling agent,
wherein the polymer having a plurality of hemocompatible groups comprises a repeating unit represented by formula (A-1): 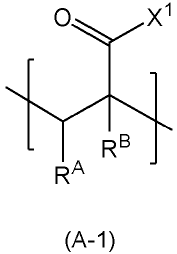 wherein:
R^{A} is selected from H and C₁₋₆ alkyl;
R^{B} is selected from H and C₁₋₆ alkyl;
X¹ is represented by formula (S-1):
-Z¹-L¹-SO₂-Y¹ (S-1)
wherein:
Z¹ is selected from O, NH and NR¹;
R¹ is selected from C₁₋₆ alkyl, C₆₋₁₀ aryl and C₆₋₁₀ aryl-C₁₋₆ alkyl;
Y' is selected from O⁻, OH, NH⁻ and NH₂;
L¹ is represented by formula (L-1):
-P¹-Q¹-W¹- (L-1)
wherein:
P¹ is selected from a single bond, C₁₋₁₀ alkylene, C₂₋₁₀ alkenylene, C₂₋₁₀ alkynylene and phenylene;
Q¹ is selected from a single bond, O, NH, NR² and phenylene; and
W¹ is selected from a single bond, C₁₋₁₀ alkylene, C₂₋₁₀ alkenylene, C₂₋₁₀ alkynylene and phenylene; and
R² is selected from C₁₋₆ alkyl, C₆₋₁₀ aryl and C₆₋₁₀ aryl-C₁₋₆ alkyl.

14. A coating according to any one of claims 1 to 9 or the coating on the medical device according to claim 10 for use in the treatment of the human or animal body by surgery or therapy and/or in a diagnostic method practised on the human or animal body.

15. A coating according to any one of claims 1 to 9, the coating on the medical device according to claim 10, or the coating for use according to claim 14, for use in reducing or preventing the clotting of blood.

## Patentansprüche

1. Beschichtung für eine medizinische Vorrichtung, umfassend:
eine Basisschicht, die ein Protein umfasst; und
eine Polymerschicht, die auf der Basisschicht angeordnet ist;
wobei:
die Polymerschicht zu der Basisschicht konjugiert ist;
die Polymerschicht ein Polymer mit einer Vielzahl von hämokompatiblen Gruppen umfasst und das Polymer mit einer Vielzahl von hämokompatiblen Gruppen mit dem Protein vernetzt ist;
jede hämokompatible Gruppe unabhängig ausgewählt ist aus einer Sulfonsäuregruppe, einer Sulfonamidgruppe, einer Sulfaminsäuregruppe, einer Hydrogensulfatgruppe und einer konjugierten Base davon, und wobei das Polymer mit einer Vielzahl von hämokompatiblen Gruppen eine Wiederholungseinheit dargestellt durch Formel (A-1) umfasst: 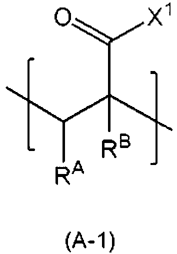
wobei:
R^{A} ausgewählt ist aus H und C₁₋₆-Alkyl;
R^{B} ausgewählt ist aus H und C₁₋₆-Alkyl;
X¹ dargestellt ist durch Formel (S-1):
-Z¹-L¹-SO₂-Y¹ (S-1)
wobei:
Z¹ ausgewählt ist aus O, NH und NR¹;
R¹ ausgewählt ist aus C₁₋₆-Alkyl, C₆₋₁₀-Aryl und C₆₋₁₀-Aryl-C₁₋₆-alkyl;
Y¹ ausgewählt ist aus O⁻, OH, NH⁻ und NH₂;
L¹ dargestellt ist durch Formel (L-1):
-P¹-Q¹-W¹- (L-1)
wobei:
P¹ ausgewählt ist aus einer Einfachbindung, C₁₋₁₀-Alkylen, C₂₋₁₀-Alkenylen, C₂₋₁₀-Alkinylen und Phenylen;
Q¹ ausgewählt ist aus einer Einfachbindung, O, NH, NR² und Phenylen; und
W¹ ausgewählt ist aus einer Einfachbindung, C₁₋₁₀-Alkylen, C₂₋₁₀-Alkenylen, C₂₋₁₀-Alkinylen und Phenylen; und
R² ausgewählt ist aus C₁₋₆-Alkyl, C₆₋₁₀-Aryl und C₆₋₁₀-Aryl-C₁₋₆-alkyl.

2. Beschichtung gemäß Anspruch 1, wobei das Polymer mit einer Vielzahl von hämokompatiblen Gruppen mit dem Protein durch Amidbindungen, die zwischen dem Polymer und dem Protein gebildet sind, vernetzt ist.

3. Beschichtung gemäß Anspruch 1 oder Anspruch 2, wobei das Protein Albumin ist, bevorzugt ein menschliches Albumin.

4. Beschichtung gemäß einem der vorhergehenden Ansprüche, wobei jede hämokompatible Gruppe ausgewählt ist aus einer Sulfonsäuregruppe oder einer Sulfonatgruppe.

5. Beschichtung gemäß einem der Ansprüche 1 bis 4, wobei:
(a) R^{A} ausgewählt ist aus H und Methyl, bevorzugt R^{A} Wasserstoff ist; und/oder
(b) R^{B} ausgewählt ist aus H und Methyl, bevorzugt R^{B} Wasserstoff ist.

6. Beschichtung gemäß einem der Ansprüche 1 bis 5, wobei:
(a) Z¹ ausgewählt ist aus O und NH, bevorzugt Z¹ NH ist; und/oder
(b) Y¹ ist ausgewählt aus O⁻ und OH.

7. Beschichtung gemäß einem der Ansprüche 1 bis 6, wobei:
(a) P¹ ausgewählt ist aus C₁₋₁₀-Alkylen, C₂₋₁₀-Alkenylen und C₂₋₁₀-Alkinylen; Q¹ eine Einfachbindung ist und W¹ eine Einfachbindung ist, bevorzugt P¹ C₁₋₁₀-Alkylen, optional C₂₋₆-Alkylen oder bevorzugter P¹ Isobutylen ist; oder
(b) P¹ ausgewählt ist aus C₁₋₁₀-Alkylen, C₂₋₁₀-Alkenylen und C₂₋₁₀-Alkinylen; Q¹ ausgewählt ist aus O, NH und NR² und W¹ eine Einfachbindung ist, bevorzugt Q¹ ausgewählt ist aus O und NH.

8. Beschichtung gemäß einem der vorhergehenden Ansprüche, wobei das Polymer mit einer Vielzahl von hämokompatiblen Gruppen eine Wiederholungseinheit dargestellt durch Formel (B-1) umfasst: 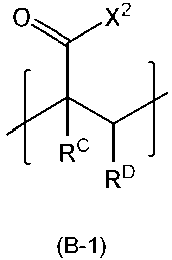 wobei:
R^{C} ausgewählt ist aus H und C₁₋₆-Alkyl;
R^{D} ausgewählt ist aus H, C₁₋₆-Alkyl und -C(O)X²;
jedes X² unabhängig ausgewählt ist aus O⁻, OH, OR³, NH₂, NHR³ und N(R³)₂; und
jedes R³ unabhängig ausgewählt ist aus optional substituiertem C₁₋₆-Alkyl, optional substituiertem C₁₋₆-Aryl und optional substituiertem C₆₋₁₀-Aryl-C₁₋₆-alkyl.

9. Beschichtung gemäß einem der vorhergehenden Ansprüche, wobei das Polymer mit einer Vielzahl von hämokompatiblen Gruppen ein Copolymer ist.

10. Medizinische Vorrichtung mit einer Oberfläche, die mit einer Beschichtung gemäß einem der Ansprüche 1 bis 9 beschichtet ist.

11. Verfahren zum Herstellen einer Beschichtung für eine medizinische Vorrichtung wie in einem der Ansprüche 1 bis 9 definiert, wobei das Verfahren Folgendes umfasst:
(a) Aufbringen eines Polymers mit einer Vielzahl von hämokompatiblen Gruppen auf eine Basisschicht, um eine Polymerschicht zu bilden,
wobei:
jede hämokompatible Gruppe unabhängig ausgewählt ist aus einer Sulfonsäuregruppe, einer Sulfonamidgruppe, einer Sulfaminsäuregruppe, einer Hydrogensulfatgruppe und einer konjugierten Base davon;
die Basisschicht ein Protein umfasst; und
das Polymer mit einer Vielzahl von hämokompatiblen Gruppen eine Wiederholungseinheit dargestellt durch Formel (A-1) umfasst: 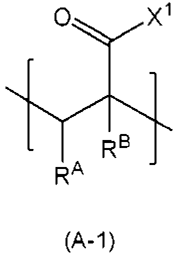
wobei:
R^{A} ausgewählt ist aus H und C₁₋₆-Alkyl;
R^{B} ausgewählt ist aus H und C₁₋₆-Alkyl;
X¹ dargestellt ist durch Formel (S-1):
-Z¹-L¹-SO₂-Y¹ (S-1)
wobei:
Z¹ ausgewählt ist aus O, NH und NR¹;
R¹ ausgewählt ist aus C₁₋₆-Alkyl, C₆₋₁₀-Aryl und C₆₋₁₀-Aryl-C₁₋₆-alkyl;
Y¹ ausgewählt ist aus O⁻, OH, NH⁻ und NH₂;
L¹ dargestellt ist durch Formel (L-1):
-P¹-Q¹-W¹- (L-1)
wobei:
P¹ ausgewählt ist aus einer Einfachbindung, C₁₋₁₀-Alkylen, C₂₋₁₀-Alkenylen, C₂₋₁₀-Alkinylen und Phenylen;
Q¹ ausgewählt ist aus einer Einfachbindung, O, NH, NR² und Phenylen; und
W¹ ausgewählt ist aus einer Einfachbindung, C₁₋₁₀-Alkylen, C₂₋₁₀-Alkenylen, C₂₋₁₀-Alkinylen und Phenylen; und
R² ausgewählt ist aus C₁₋₆-Alkyl, C₆₋₁₀-Aryl und C₆₋₁₀-Aryl-C₁₋₆-alkyl;
(b) Koppeln des Polymers mit einer Vielzahl von hämokompatiblen Gruppen an das Protein unter Verwendung eines Kopplungsmittels.

12. Verfahren nach Anspruch 11, wobei das Kopplungsmittel ein Carbodiimid-Kreuzkopplungsmittel ist.

13. Kit zum Beschichten einer medizinischen Vorrichtung mit einer Beschichtung wie in einem der Ansprüche 1 bis 9 definiert, wobei das Kit Folgendes umfasst:
(a) ein Polymer mit einer Vielzahl von hämokompatiblen Gruppen, wobei jede hämokompatible Gruppe unabhängig ausgewählt ist aus einer Sulfonsäuregruppe, einer Sulfonamidgruppe, einer Sulfaminsäuregruppe, einer Hydrogensulfatgruppe und einer konjugierten Base davon,
(b) eine Basisbeschichtungslösung, die ein Protein umfasst; und
(c) ein Kopplungsmittel,
wobei das Polymer mit einer Vielzahl von hämokompatiblen Gruppen eine Wiederholungseinheit dargestellt durch Formel (A-1) umfasst: 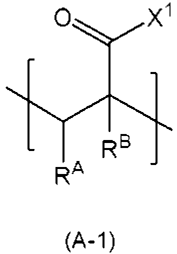 wobei:
R^{A} ausgewählt ist aus H und C₁₋₆-Alkyl;
R^{B} ausgewählt ist aus H und C₁₋₆-Alkyl;
X¹ dargestellt ist durch Formel (S-1):
-Z¹-L¹-SO₂-Y¹ (S-1)
wobei:
Z¹ ausgewählt ist aus O, NH und NR¹;
R¹ ausgewählt ist aus C₁₋₆-Alkyl, C₆₋₁₀-Aryl und C₆₋₁₀-Aryl-C₁₋₆-alkyl;
Y¹ ausgewählt ist aus O⁻, OH, NH⁻ und NH₂;
L¹ dargestellt ist durch Formel (L-1):
-P¹-Q¹-W¹- (L-1)
wobei:
P¹ ausgewählt ist aus einer Einfachbindung, C₁₋₁₀-Alkylen, C₂₋₁₀-Alkenylen, C₂₋₁₀-Alkinylen und Phenylen;
Q¹ ausgewählt ist aus einer Einfachbindung, O, NH, NR² und Phenylen; und
W¹ ausgewählt ist aus einer Einfachbindung, C₁₋₁₀-Alkylen, C₂₋₁₀-Alkenylen, C₂₋₁₀-Alkinylen und Phenylen; und
R² ausgewählt ist aus C₁₋₆-Alkyl, C₆₋₁₀-Aryl und C₆₋₁₀-Aryl-C₁₋₆-alkyl.

14. Beschichtung gemäß einem der Ansprüche 1 bis 9 oder Beschichtung auf der medizinischen Vorrichtung gemäß Anspruch 10 zur Verwendung bei der Behandlung des menschlichen oder tierischen Körpers durch Operation oder Therapie und/oder in einem Diagnoseverfahren, das an dem menschlichen oder tierischen Körper praktiziert wird.

15. Beschichtung gemäß einem der Ansprüche 1 bis 9, Beschichtung auf der medizinischen Vorrichtung gemäß Anspruch 10 oder Beschichtung zur Verwendung gemäß Anspruch 14 zur Verwendung beim Reduzieren oder Verhindern der Gerinnung von Blut.

## Revendications

1. Revêtement destiné à un dispositif médical comprenant :
une couche de base comprenant une protéine ; et
une couche polymère disposée sur la couche de base :
ladite couche polymère étant conjuguée à la couche de base ;
ladite couche polymère comprenant un polymère comportant une pluralité de groupes hémocompatibles, et ledit polymère comportant une pluralité de groupes hémocompatibles étant réticulé à la protéine ;
chaque groupe hémocompatible étant indépendamment choisi parmi un groupe acide sulfonique, un groupe sulfonamide, un groupe acide sulfamique, un groupe hydrogénosulfate et une base conjuguée de ceux-ci, et ledit polymère comportant une pluralité de groupes hémocompatibles comprenant un motif récurrent représenté par la formule (A-1) : 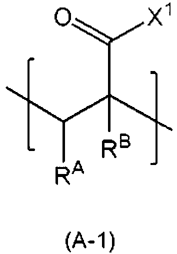 dans laquelle :
R^{A} est choisi parmi un atome H et un groupe C₁₋₆ alkyle ;
R^{B} est choisi parmi un atome H et un groupe C₁₋₆ alkyle ;
X¹ est représenté par la formule (S-1) :
-Z¹-L¹-SO₂-Y¹ (S-1)
dans laquelle :
Z¹ est sélectionné parmi un atome O, les groupes NH et NR¹ ;
R¹ est choisi parmi les groupes C₁₋₆ alkyle, C₆₋₁₀ aryle et C₆₋₁₀ aryl-C₁₋₆ alkyle ;
Y¹ est sélectionné parmi un atome O⁻, les groupes OH, NH⁻ et NH₂ ;
L¹ est représenté par la formule (L-1) :
-P¹-Q¹-W¹- (L-1)
dans laquelle :
P¹ est choisi parmi une liaison simple, les groupes C₁₋₁₀ alkylène, C₂₋₁₀ alcénylène, C₂₋₁₀ alcynylène et phénylène ;
Q¹ est choisi parmi une liaison simple, un atome O, les groupes NH, NR² et phénylène ; et
W¹ est choisi parmi une liaison simple, les groupes C₁₋₁₀ alkylène, C₂₋₁₀ alcénylène, C₂₋₁₀ alcynylène et phénylène ; et
R² est choisi parmi les groupes C₁₋₆ alkyle, C₆₋₁₀ aryle et C₆₋₁₀ aryl-C₁₋₆ alkyle.

2. Revêtement selon la revendication 1, ledit polymère comportant une pluralité de groupes hémocompatibles étant réticulé à la protéine par des liaisons amide formées entre le polymère et la protéine.

3. Revêtement selon la revendication 1 ou la revendication 2, ladite protéine étant de l'albumine, de préférence une albumine humaine.

4. Revêtement selon l'une quelconque des revendications précédentes, chaque groupe hémocompatible étant choisi parmi un groupe acide sulfonique ou un groupe sulfonate.

5. Revêtement selon l'une quelconque des revendications 1 à 4 :
(a) R^{A} étant choisi parmi un atome H et un groupe méthyle, de préférence R^{A} représentant un atome d'hydrogène ; et/ou
(b) R^{B} étant choisi parmi un atome H et un groupe méthyle, de préférence R^{B} représentant un atome d'hydrogène.

6. Revêtement selon l'une quelconque des revendications 1 à 5 :
(a) Z¹ étant choisi parmi un atome O et un groupe NH, de préférence Z¹ représentant un groupe NH ; et/ou
(b) Y¹ étant choisi parmi un atome O⁻ et un groupe OH.

7. Revêtement selon l'une quelconque des revendications 1 à 6 :
(a) P¹ étant choisi parmi les groupes C₁₋₁₀ alkylène, C₂₋₁₀ alcénylène et C₂₋₁₀ alcynylène ; Q¹ représentant une liaison simple et W¹ représentant une liaison simple, de préférence P¹ représentant un groupe C₁₋₁₀ alkylène, éventuellement un groupe C₂₋₆ alkylène, ou plus préférablement P¹ représentant un groupe iso-butylène ; ou
(b) P¹ étant choisi parmi les groupes C₁₋₁₀ alkylène, C₂₋₁₀ alcénylène et C₂₋₁₀ alcynylène ; Q¹ étant choisi parmi un atome O, les groupes NH et NR² et W¹ représentant une liaison simple, de préférence Q¹ étant choisi parmi un atome O et un groupe NH.

8. Revêtement selon l'une quelconque des revendications précédentes, ledit polymère comportant une pluralité de groupes hémocompatibles comprenant un motif récurrent représenté par la formule (B-1) : 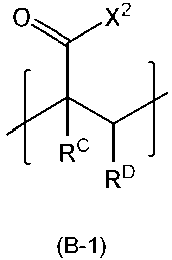 dans laquelle :
R^{C} est choisi parmi un atome H et un groupe C₁₋₆ alkyle ;
R^{D} est choisi parmi un atome H, les groupes C₁₋₆ alkyle et -C(O)X² ;
chaque X² est indépendamment choisi parmi un atome O⁻, les groupes OH, OR³, NH₂, NHR³ et N(R³)₂ ; et
chaque R³ est indépendamment choisi parmi un groupe C₁₋₆ alkyle éventuellement substitué, un groupe C₁₋₆ aryle éventuellement substitué et un groupe C₆₋₁₀ aryl-C₁₋₆ alkyle éventuellement substitué.

9. Revêtement selon l'une quelconque des revendications précédentes, ledit polymère comportant une pluralité de groupes hémocompatibles étant un copolymère.

10. Dispositif médical possédant une surface revêtue d'un revêtement selon l'une quelconque des revendications 1 à 9.

11. Procédé de fabrication d'un revêtement destiné à un dispositif médical selon l'une quelconque des revendications 1 à 9, le procédé comprenant :
(a) l'application d'un polymère comportant une pluralité de groupes hémocompatibles sur une couche de base de manière à former une couche polymère :
chaque groupe hémocompatible étant indépendamment choisi parmi un groupe acide sulfonique, un groupe sulfonamide, un groupe acide sulfamique, un groupe hydrogénosulfate et une base conjuguée de ceux-ci ;
ladite couche de base comprenant une protéine ; et
ledit polymère comportant une pluralité de groupes hémocompatibles comprenant un motif récurrent représenté par la formule (A-1) : 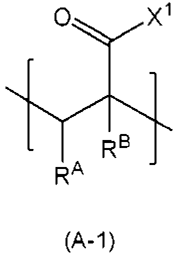 dans laquelle :
R^{A} est choisi parmi un atome H et un groupe C₁₋₆ alkyle ;
R^{B} est choisi parmi un atome H et un groupe C₁₋₆ alkyle ;
X¹ est représenté par la formule (S-1) :
-Z¹-L¹-SO₂-Y¹ (S-1)
dans laquelle :
Z¹ est sélectionné parmi un atome O, les groupes NH et NR¹ ;
R¹ est choisi parmi les groupes C₁₋₆ alkyle, C₆₋₁₀ aryle et C₆₋₁₀ aryl-C₁₋₆ alkyle ;
Y¹ est sélectionné parmi un atome O⁻, les groupes OH, NH⁻ et NH₂ ;
L¹ est représenté par la formule (L-1) :
-P¹-Q¹-W¹- (L-1)
dans laquelle :
P¹ est choisi parmi une liaison simple, les groupes C₁₋₁₀ alkylène, C₂₋₁₀ alcénylène, C₂₋₁₀ alcynylène et phénylène ;
Q¹ est choisi parmi une liaison simple, un atome O, les groupes NH, NR² et phénylène ; et
W¹ est choisi parmi une liaison simple, les groupes C₁₋₁₀ alkylène, C₂₋₁₀ alcénylène, C₂₋₁₀ alcynylène et phénylène ; et
R² est choisi parmi les groupes C₁₋₆ alkyle, C₆₋₁₀ aryle et C₆₋₁₀ aryl-C₁₋₆ alkyle ;
(b) le couplage du polymère comportant une pluralité de groupes hémocompatibles à la protéine à l'aide d'un agent de couplage.

12. Procédé selon la revendication 11, ledit agent de couplage étant un agent de couplage croisé carbodiimide.

13. Kit destiné à revêtir un dispositif médical d'un revêtement tel que défini dans l'une quelconque des revendications 1 à 9, ledit kit comprenant :
(a) un polymère comportant une pluralité de groupes hémocompatibles, chaque groupe hémocompatible étant indépendamment choisi parmi un groupe acide sulfonique, un groupe sulfonamide, un groupe acide sulfamique, un groupe hydrogénosulfate et une base conjuguée de ceux-ci,
(b) une solution de revêtement de base comprenant une protéine ; et
(c) un agent de couplage,
ledit polymère comportant une pluralité de groupes hémocompatibles comprenant un motif récurrent représenté par la formule (A-1) : 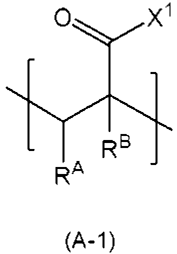 dans laquelle :
R^{A} est choisi parmi un atome H et un groupe C₁₋₆ alkyle ;
R^{B} est choisi parmi un atome H et un groupe C₁₋₆ alkyle ;
X¹ est représenté par la formule (S-1) :
-Z¹-L¹-SO₂-Y¹ (S-1)
dans laquelle :
Z¹ est sélectionné parmi un atome O, les groupes NH et NR¹ ;
R¹ est choisi parmi les groupes C₁₋₆ alkyle, C₆₋₁₀ aryle et C₆₋₁₀ aryl-C₁₋₆ alkyle ;
Y¹ est sélectionné parmi un atome O⁻, les groupes OH, NH⁻ et NH₂ ;
L¹ est représenté par la formule (L-1) :
-P¹-Q¹-W¹- (L-1)
dans laquelle :
P¹ est choisi parmi une liaison simple, les groupes C₁₋₁₀ alkylène, C₂₋₁₀ alcénylène, C₂₋₁₀ alcynylène et phénylène ;
Q¹ est choisi parmi une liaison simple, un atome O, les groupes NH, NR² et phénylène ; et
W¹ est choisi parmi une liaison simple, les groupes C₁₋₁₀ alkylène, C₂₋₁₀ alcénylène, C₂₋₁₀ alcynylène et phénylène ; et
R² est choisi parmi les groupes C₁₋₆ alkyle, C₆₋₁₀ aryle et C₆₋₁₀ aryl-C₁₋₆ alkyle.

14. Revêtement selon l'une quelconque des revendications 1 à 9 ou revêtement sur le dispositif médical selon la revendication 10 destiné à être utilisé dans le traitement du corps humain ou animal par une chirurgie ou une thérapie et/ou dans un procédé de diagnostic pratiqué sur le corps humain ou animal.

15. Revêtement selon l'une quelconque des revendications 1 à 9, revêtement sur le dispositif médical selon la revendication 10, ou revêtement destiné à être utilisé selon la revendication 14, destiné à être utilisé pour réduire ou prévenir la coagulation du sang.
